# EUROPÄISCHE PATENTSCHRIFT

(11) **EP 0 381 795 B1**
(45) Veröffentlichungstag und Bekanntmachung des Hinweises auf die Patenterteilung: **16.06.1993**
(21) Anmeldenummer: 89102329.3
(22) Anmeldetag: 10.02.1989
(51) Int. Cl.: A61B 6/04, A61B 6/08, A61B 6/10, A61B 6/00

(54) **Röntgendiagnostikvorrichtung**
X-ray diagnostic apparatus
Appareil de diagnostic aux rayons X

(43) Veröffentlichungstag der Anmeldung: 16.08.1990
(73) Patentinhaber: SIEMENS AKTIENGESELLSCHAFT, 80333 München (DE)
(72) Erfinder: Hubert, Günter, D-8523 Baiersdorf (DE); Seyler, Gerhard, Dipl-Ing. (FH), D-8526 Bubenreuth (DE); Wessels, Gerd, Dr.Dipl.Ing., D-8520 Erlangen (DE)

(56) Entgegenhaltungen:
- DE-A- 2 438 708
- DE-A- 2 550 437
- DE-A- 2 608 452
- DE-B- 2 321 968
- GB-A- 856 797
- GB-A- 1 200 814

## Beschreibung

Die Erfindung betrifft eine Röntgendiagnostikvorrichtung mit einer Patientenliege sowie einem Aufnahmegerät für Röntgenaufnahmen mit einer unter dem Patienten anzuordnenden Röntgenfilmkassette.

Eine Röntgendiagnostikvorrichtung dieser Art wird z.B. in einer Intensivstation zur Anfertigung von Röntgenaufnahmen verwendet. Es ist bekannt, den Röntgenstrahler an einem fahrbaren Stativ anzuordnen, das zur Anfertigung von Röntgenaufnahmen an die Patientenliege herangefahren wird. Der Patient muß vom Stationspersonal hochgehoben werden, damit die Röntgenfilmkassette zwischen Patient und Patientenliege eingelegt werden kann. Anschließend wird der Röntgenstrahler über die Patientenliege gebracht.

Störend ist dabei, daß für die Anfertigung von Röntgenaufnahmen ein Hochheben des Patienten erforderlich ist. Ferner bereitet die exakte Zentrierung und Einblendung der Röntgenstrahlung Schwierigkeiten.

In der GB-A-1 200 814 ist eine Röntgendiagnostikvorrichtung mit einer Patientenliege sowie einem Kassettenhalter für eine unter dem Patienten anzuordnende Röntgenfilmkassette beschrieben. Die Patientenliege weist eine obere Tragfläche für den Patienten auf. Unter dieser Tragfläche ist der Kassettenhalter in ein Tischoberteil einschiebbar. Das ortsfeste Tischoberteil dient dabei nicht unmittelbar zum Tragen des Patienten.

Der Erfindung liegt die Aufgabe zugrunde, eine Röntgendiagnostikvorrichtung der eingangs genannten Art so auszubilden, daß für die Anfertigung von Röntgenaufnahmen ein Hochheben des Patienten nicht erforderlich und eine einfache und genaue Zentrierung der Röntgenstrahlung in bezug auf die Röntgenfilmkassette möglich ist.

Diese Aufgabe ist erfindungsgemäß dadurch gelöst, daß die Patientenliege zwei Patienten-Tragflächen aufweist, von denen die obere Tragfläche für Röntgenstrahlen durchlässig ist und die untere Tragfläche in vertikaler Richtung verstellbar und so weit absenkbar ist, daß die Röntgenfilmkassette zur Röntgenaufnahme zwischen beiden Tragflächen einschiebbar ist. Bei der erfindungsgemäßen Röntgendiagnostikvorrichtung kann bei auf der oberen Tragfläche liegendem Patienten ein Absenken der unteren Tragfläche erfolgen, so daß die Röntgenfilmkassette, die vorzugsweise in einem Kassettenhalter liegt, der an einem Wagen befestigt ist, leicht unter dem Patienten eingeschoben werden kann. Dadurch ist auch eine einfache und genaue Ausrichtung der Röntgenfilmkassette möglich.

Einzelheiten der Erfindung ergeben sich aus den abhängigen Ansprüchen.

Die Erfindung ist nachfolgend anhand eines in der Zeichnung dargestellten Ausführungsbeispieles näher erläutert. Es zeigen:
- Fig. 1 und 2: die Patientenliege einer Röntgendiagnostikvorrichtung nach der Erfindung in zwei verschiedenen Stellungen, und
- Fig. 3: das der Patientenliege gemäß den Fig. 1 und 2 zugeordnete Aufnahmegerät.

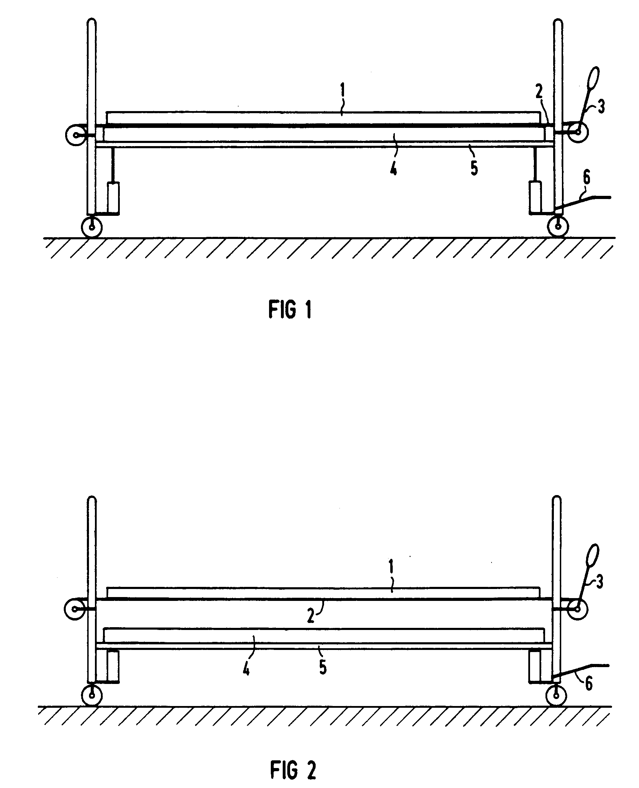

In den Fig. 1 und 2 ist eine Patientenliege dargestellt, die als Patientenauflage eine Schaumstoffmatte 1 auf einem Spanntuch 2 aufweist. Das Spanntuch 2 ist mit Hilfe eines Spannhebels 3 spannbar. Unterhalb des Spanntuches 2 liegt eine Matratze 4 auf einer Auflage 5, welche mit Hilfe eines Hebels 6 hydraulisch höhenverstellbar ist.

Die Schaumstoffmatte 1 und das Spanntuch 2 sind für Röntgenstrahlen durchlässig. Liegt ein Patient auf der Patientenliege gemäß Fig. 1 und soll eine Röntgenaufnahme angefertigt werden, so wird das Spanntuch 2 mit Hilfe des Hebels 3 gespannt. Danach wird die Matratze 4 mit der Auflage 5 in die in der Fig. 2 dargestellte Stellung abgesenkt. Danach kann in den freien Raum zwischen dem Spanntuch 2 und der Matratze 4 eine Röntgenfilmkassette eingeschoben werden. Der Patient braucht dabei nicht bewegt zu werden. Nach der Röntgenaufnahme wird die Matratze 4 mit der Auflage 5 wieder hochgefahren und das Spanntuch 2 mit Hilfe des Hebels 3 entspannt, so daß der Patient wieder auf der Matratze 4 aufliegt.

Anstelle der in den Fig. 1 und 2 dargestellten hydraulischen Verstellung der Matratze 4 mit der Auflage 5 ist es auch möglich, andere Verstellmittel, z.B. einen Kniehebelmechanismus oder Elektromotoren, vorzusehen.

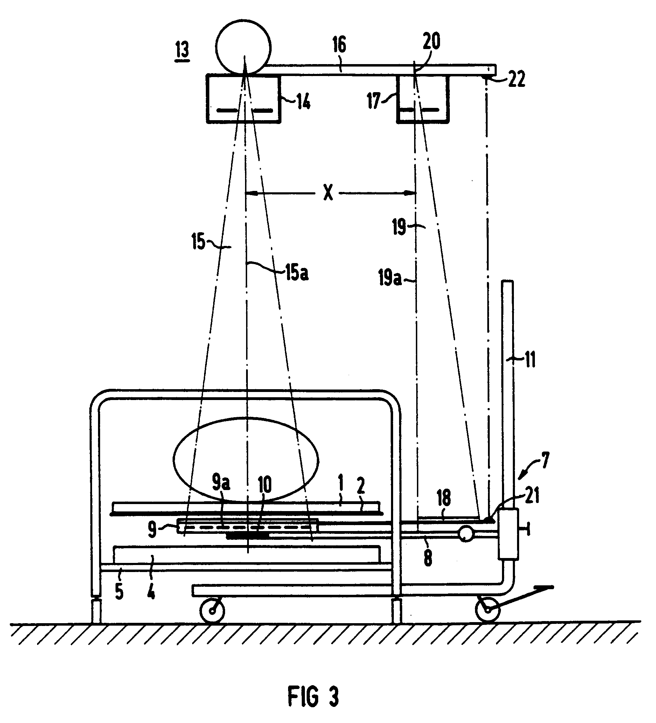

Die Fig. 3 zeigt die Patientenliege in der in der Fig. 2 gezeigten Stellung in einer Stirnansicht. Teile, die mit den Teilen der Fig. 1 und 2 gleich sind, sind mit den gleichen Bezugszeichen bezeichnet. In der Fig. 3 ist ein Wagen 7 dargestellt, der einen Kassettenhalter 8 trägt, der mit einem Kassettenfach 9 für eine Röntgenfilmkassette 9a versehen ist. Hinter dem Kassettenfach 9 liegt ein Röntgenstrahlendetektor 10. Der Kassettenhalter 8 ist an einem Stativ 11 des Wagens 7 höhenverstellbar gelagert. Er wird in der richtigen Höhe zwischen dem Spanntuch 2 und der Matratze 4 eingeschoben.

Zur Ausrichtung der Röntgenfilmkassette in bezug auf das von einem Röntgenstrahler 13 mit einer Primärstrahlenblende 14 ausgehende Röntgenstrahlenbündel 15 ist an einem Ausleger 16 des Röntgenstrahlers 13 eine Strahlenblende 17 angeordnet, die ein optisches Fadenkreuz in einem Justierfeld 18 des Kassettenhalters 8 erzeugt. Das Justierfeld 18 liegt so weit seitlich neben dem Kassettenfach 9, daß die Lichtstrahlung seitlich an der Patientenliege vorbei verläuft. Im Justierfeld 18 sind Markierungen angebracht, die mit dem Fadenkreuz zur Deckung gebracht werden. Dann ist die Röntgenfilmkassette 9a im Kassettenfach 9 auf das Aufnahmefeld ausgerichtet. Hierzu wird die Strahlenblende 17 synchron mit der Primärstrahlenblende 14 verstellt. Das Lichtstrahlenbündel 19 wird von einer Lichtquelle 20 erzeugt.

Der Abstand X der Strahlenblende 17 von der Primärstrahlenblende 14 ist der Abstand des in der Fig. 3 vertikal verlaufenden Lichtstrahles 19a vom dazu parallelen Zentralstrahl 15a des Röntgenstrahlenbündels 15. Die Strahlenblende 17 bildet nur ein halbes Fadenkreuz, während durch die Primärstrahlenblende 14 das Aufnahmefeld mit einem Lichtvisier mit einem vollen optischen Fadenkreuz am Patienten ausgeleuchtet werden kann. Mit dem halben Fadenkreuz und halber Kassettengröße auf dem Justierfeld 18 kann der Kassettenwagen genau parallel zur Patientenliege und senkrecht zum Zentralstrahl 15a ausgerichtet werden. Die gewünschte Kassettengröße kann ebenfalls in der Kassettenebene exakt eingeblendet werden. Dabei wird im Justierfeld 18 nur die halbe Kassettengröße wiedergegeben.

Nach der Einstellung wird der Wagen 7 arretiert. Der Kassettenhalter 8 kann für andere Kassettengrößen mit zugehörigen Streustrahlenrastern leicht ausgetauscht werden.

Die Dosismessung erfolgt über den Röntgenstrahlendetektor 10, dessen elektrische Signale mit Hilfe eines Infrarotsenders 21 drahtlos übertragen und von einem Empfänger 22 am Ausleger 16 des Röntgenstrahlers 13 zur Steuerung eines Belichtungsautomaten empfangen werden. Die Stromversorgung kann durch einen im Wagen 7 eingebauten Akku gewährleistet sein, der bei Nichtbenutzung des Aufnahmegerätes an der Steckdose wieder aufgeladen wird.

Aus der Fig. 3 geht hervor, daß der Wagen 7 völlig unabhängig vom Röntgenstrahler 13 verstellbar ist. Trotzdem ist eine einfache und sichere Ausrichtung der Kassette im Kassettenfach 9 auf das Röntgenstrahlenbündel 15 sichergestellt. Der Röntgenstrahler 13 ist mit Hilfe des Auslegers 16 an einem weiteren Wagen oder an einem dreidimensional verstellbaren Deckenstativ befestigt, der die dem Röntgenstrahler 13 zugeordneten Komponenten aufweist.

## Patentansprüche

1. Röntgendiagnostikvorrichtung mit einer Patientenliege sowie einem Aufnahmegerät für Röntgenaufnahmen mit einer unter dem Patienten anzuordnenden Röntgenfilmkassette, **dadurch gekennzeichnet,** daß die Patientenliege zwei Patienten-Tragflächen (1, 4) aufweist, von denen die obere Tragfläche (1) für Röntgenstrahlen durchlässig ist und die untere Tragfläche (4) in vertikaler Richtung verstellbar und soweit absenkbar ist, daß die Röntgenfilmkassette (9a) zur Röntgenaufnahme zwischen beiden Tragflächen (1, 4) einschiebbar ist.

2. Röntgendiagnostikvorrichtung nach Anspruch 1, **dadurch gekennzeichnet,** daß das Aufnahmegerät von einem Wagen (7) gebildet ist, der einen Kassettenhalter (8) aufweist.

3. Röntgendiagnostikvorrichtung nach Anspruch 2, **dadurch gekennzeichnet,** daß an einem Ausleger (16) des Röntgenstrahlers (13) ein Lichtsender (20) angeordnet ist, dessen Lichtbündel (19) auf einem Justierfeld (18) am Kassettenhalter (8) neben dem Kassettenfach (9) auftrifft.

4. Röntgendiagnostikvorrichtung nach einem der Ansprüche 1 bis 3, **dadurch gekennzeichnet,** daß ein Belichtungsautomat vorhanden ist, bei dem die Detektorsignale drahtlos zu einem Empfänger (22) übertragen werden.

5. Röntgendiagnostikvorrichtung nach Anspruch 4, **dadurch gekennzeichnet,** daß am Kassettenhalter (8) ein Sender (12) für die drahtlose Übertragung der Signale eines Röntgenstrahlendetektors (10) zu dem am Ausleger (16) des Röntgenstrahlers (15) angeordneten Empfängers (22) vorgesehen ist.

6. Röntgendiagnostikvorrichtung nach einem der Ansprüche 1 bis 5, **dadurch gekennzeichnet,** daß die obere Tragfläche von einem spannbaren und entspannbaren Spanntuch (1) gebildet ist.

## Claims

1. X-ray diagnostic device having a patient rest and a recording unit for X-ray photographs with an X-ray film cassette to be arranged under the patient, characterized in that the patient rest has two patient supporting surfaces (1, 4), the upper supporting surface (1) of which is penetrable by X-rays and the lower supporting surface (4) can be adjusted in the vertical direction and can be lowered so far that the X-ray film cassette (9a) for the X-ray photograph can be inserted between the two supporting surfaces (1, 4).

2. X-ray diagnostic device according to claim 1, characterized in that the recording unit is formed by a carriage (7) which has a cassette holder (8).

3. X-ray diagnostic device according to claim 2, characterized in that a light transmitter (20) is arranged on a bracket (16) of the X-ray source (13), the light beam (19) of which light transmitter strikes an adjustment field (18) on the cassette holder (8) next to the cassette compartment (9).

4. X-ray diagnostic device according to one of claims 1 to 3, characterized in that an automatic exposure device is present, with which detector signals are transmitted by radio to a receiver (22).

5. X-ray diagnostic device according to claim 4, characterized in that a transmitter (12) for the radio transmission of the signals of an X-ray detector (10) to the receiver (22) arranged on the bracket (16) of the X-ray source (15) is provided on the cassette holder (8).

6. X-ray diagnostic device according to one of claims 1 to 5, characterized in that the upper supporting surface is formed by a stretched cloth (1) which can be stretched and slackened.

## Revendications

1. Appareil de radiodiagnostic comportant une couchette pour patient ainsi qu'un dispositif d'enregistrement de radiographies comportant une cassette pour film radiographique, devant être disposée sous le patient, caractérisé par le fait que la couchette pour patient comporte deux surfaces de support (1,4) pour le patient, parmi lesquelles la surface de support supérieure (1) est transparente aux rayons X et la surface de support inférieure (4) est déplaçable verticalement et peut être abaissée au point que la cassette (9a) pour film radiographique peut être insérée entre les deux surfaces de support (1,4) pour la réalisation d'une radiographie.

2. Appareil de radiodiagnostic suivant la revendication 1, caractérisé par le fait que le dispositif d'enregistrement est formé par un chariot (7), qui comporte un porte-cassette (8).

3. Appareil de radiodiagnostic suivant la revendication 2, caractérisé par le fait que sur un bras en console (16) de l'émetteur radiologique (13) est montée une source de lumière (20), dont le faisceau de lumière (19) rencontre une zone d'ajustement (18) situé sur le porte-cassette (8) à côté du casier (9) de logement de la cassette.

4. Appareil de radiodiagnostic suivant l'une des revendications 1 à 3, caractérisé par le fait qu'il est prévu un dispositif automatique d'exposition, dans lequel les signaux du détecteur sont transmis sans fil à un récepteur (22).

5. Appareil de radiodiagnostic suivant la revendication 4, caractérisé par le fait que sur le porte-cassette (8) il est prévu un émetteur (12) servant à réaliser la transmission sans fil des signaux d'un détecteur (10) de rayons X au récepteur (22) disposé sur le bras en console (16) de l'émetteur radiologique (15).

6. Appareil de radiodiagnostic suivant l'une des revendications 1 à 5, caractérisé par le fait que la surface de support supérieure est formée par une toile de serrage (1) pouvant être tendue et détendue.
